# EUROPÄISCHE PATENTSCHRIFT

(11) **EP 2 419 038 B1**
(45) Veröffentlichungstag und Bekanntmachung des Hinweises auf die Patenterteilung: **16.01.2013**
(21) Anmeldenummer: 10711580.0
(22) Anmeldetag: 26.03.2010
(51) Int. Cl.: A61B 18/14, A61B 17/3203, A61B 17/00

(54) **ENDOSKOPISCHES CHIRURGIEINSTRUMENT**
ENDOSCOPIC SURGICAL INSTRUMENT
INSTRUMENT CHIRURGICAL ENDOSCOPIQUE

(30) Priorität: 16.04.2009 DE 102009017636
(43) Veröffentlichungstag der Anmeldung: 22.02.2012
(73) Patentinhaber: ERBE Elektromedizin GmbH, 72072 Tübingen (DE)
(72) Erfinder: KARWEI, Dietmar, 72131 Ofterdingen (DE)
(74) Vertreter: Rüger, Barthelt & Abel
(86) Internationale Anmeldenummer: PCT/EP2010/001942
(87) Internationale Veröffentlichungsnummer: WO 2010/118818

(56) Entgegenhaltungen:
- EP-A1- 1 522 269
- EP-A1- 1 929 968
- WO-A1-2005/104965
- WO-A1-2006/108480
- US-A1- 2004 210 284

## Beschreibung

Die Erfindung betrifft ein endoskopisches Chirurgieinstrument nach dem Oberbegriff des Patentanspruchs 1.

Insbesondere handelt es sich hierbei um ein endoskopisches, kombiniertes Chirurgieinstrument für die Wasserstrahl- und Hochfrequenzchirurgie, insbesondere für die endoskopische Mukosa-Elevation und -Resektion.

Die Resektion großflächiger Tumore im Gastrointestinaltrakt, die auf die Mukosa begrenzt sind, soll möglichst in einer Sitzung und möglichst vollständig erfolgen. Dazu wird üblicherweise eine Schlingen- oder Kappentechnik eingesetzt, wobei allein abhängig vom Schlingen- bzw. Kappendurchmesser unterschiedliche große, im Wesentlichen kreisrunde Resektate erzielt werden. Großflächige Tumore mit einem Durchmesser größer als 8 cm können nur in Teilschritten abgetragen werden.

Zur Durchführung einer derartigen vollständigen Operation wird in der DE 10 2005 038 694 A1 ein endoskopisches Instrument vorgeschlagen, das eine Vorrichtung zur Wasserstrahlchirurgie mit einer Vorrichtung zur Hochfrequenzchirurgie zur einheitlichen Handhabung kombiniert. Dadurch wird ein Multifunktionsinstrument bereitgestellt, das die Vorteile der Wasserstrahlchirurgie mit jenen der Hochfrequenzchirurgie verbinden soll. Das Instrument ermöglicht das dosierte Unterspritzen der Mukosa mit einer NaCl-Lösung, um diese von der Muskularis abzuheben. Weiterhin ist mit diesem Instrument auch ein Trennen mit dem Fluidstrahl möglich. Um die Submukosa zu trennen, wird die Vorrichtung zur Hochfrequenzchirurgie eingesetzt. So können mit ein und demselben Instrument zwei Arbeitsschritte hintereinander und ohne Instrumentenwechsel durchgeführt werden. Der Aufbau des bekannten Instruments sieht zwei, vom proximalen bis zum distalen - Ende des Instruments reichende Kanäle vor und zwar einen ersten Kanal für das Schneidfluid und einen zweiten Kanal, der das Hochfrequenzchirurgieinstrument verschiebbar beherbergt. Beide Kanäle werden von einer gemeinsamen Schutzhülle ummantelt und bilden so eine Einheit. Das distale Ende des ersten Kanals weist eine Düse auf, um den Fluidstrahl mit ausreichender Geschwindigkeit /Energie ausstoßen zu können, um die besagte Schneidwirkung erzielen zu können.

Nachteilig an dem bekannten Instrument ist es, dass während der Operation mit zwei am distalen Ende hervorstehenden, verschiedenen Instrumenten gearbeitet werden muss, die zwar in einem Kombinationsinstrument kombiniert vorliegen, die aber doch jeweils der vollen Aufmerksamkeit des Chirurgen bedürfen. Ferner ist das bekannte Instrument in der Herstellung aufwändig, weil für die Düse der Wasserstrahlchirurgievorrichtung einerseits und Hochfrequenzchirurgievorrichtung andererseits jeweils eigene "Instrumente" nötig sind.

Aus der EP 0 280 972 A1 ist ein Chirurgieinstrument für die offene Chirurgie bekannt, nämlich ein Handstück für den chirurgischen Einsatz, das eine Kombination aus einem Fluidstrahlschneid- sowie einem Hochfrequenzkoagulationsinstrument aufweist, um beim Schneiden mit dem Fluidstrahl auftretende blutende Gefäße sogleich mittels einem Instrument mittels hochfrequenten Stroms zu koagulieren, also verschließen zu können. Hierzu weist das Handstück eine Koagulationselektrode an seinem distalen Ende, die zugleich eine Auslassdüse für die Schneidflüssigkeit beherbergt, auf. Zum einen ist ein endoskopisches Arbeiten mit diesem Instrument nicht möglich, da eine ungewollte Berührung von Gewebe innerhalb einer Körperhöhle nicht vermieden werden kann. Darüber hinaus ist ein Schneiden mit hochfrequentem Strom ebenfalls nicht möglich. Weiterhin ist es nicht möglich, mit diesem Instrument geplante Schnittränder vor der eigentlichen Dissektion zu markieren, so dass Planung und Aufzeichnung einerseits und Schnitt andererseits zur größeren Sicherheit in zwei Phasen erfolgen müssen.

Ein endoskopisches Chirurgie instrument mit Flüssigkeitsstrahleintichtung ist aus der WO 2006/108480 bekannt.

Ausgehend vom oben genannten Stand der Technik ist es Aufgabe der vorliegenden Erfindung, ein endoskopisches Chirurgieinstrument aufzuzeigen, das einfach aufgebaut und einfach handzuhaben ist, so dass insbesondere eine Mukosa-Elevation und -Resektion in einem einzigen Arbeitsgang durchführbar ist. Weiterhin ist es Aufgabe der Erfindung, ein Herstellungsverfahren für ein solches Instrument anzugeben.

Diese Aufgabe wird durch ein endoskopisches Chirurgieinstrument nach Anspruch 1 bzw. ein Verfahren nach Anspruch 12 gelöst.

Insbesondere wird die Aufgabe durch ein endoskopisches Chirurgieinstrument gelöst, umfassend eine Flüssigkeitsstrahleinrichtung, umfassend einen Rohrabschnitt mit einer Düse an einem distalen Ende für eine Dissektion und/oder nadellose Injektion mittels einer Flüssigkeit und eine Elektrodeneinrichtung zum Schneiden und/oder Koagulieren eines Gewebes mittels HF-Strom, wobei der Rohrabschnitt die Elektrodeneinrichtung bildet und am distalen Ende des Rohrabschnittes eine Isoliereinrichtung derart angebracht ist, dass das Gewebe nur mit einem Umfangsbereich und nicht mit dem distalen Ende des Rohrabschnittes in elektrisch leitende Berührung bringbar ist.

Ein wesentlicher Punkt der Erfindung liegt darin, dass einerseits ein Teil der für die Wasserstrahlchirurgie notwendigen Leitung als HF-chirurgisches Instrument ausgebildet ist und zwar (auch) zum Schneiden, wobei nicht das distale Ende (wie beim Gegenstand nach der EP 0 280 972 A1) zum Einsatz kommt, sondern der Außenmantel des Rohrabschnittes. Um dies sicherzustellen, ist das distale Ende des Leitungs- oder Rohrabschnittes gegen eine elektrisch leitende Berührung von Gewebe durch die Isoliereinrichtung abgesichert, so dass auch in beengtem Raum bei einer endoskopischen Operation eine Minimierung der Operationsrisiken sichergestellt ist.

Vorzugsweise ist die Isoliereinrichtung als mechanische Präparationseinrichtung zum Präparieren von Gewebe ausgebildet. Es könnte die Isoliereinrichtung hakenförmig ausgebildet sein, jedoch genügt es im Allgemeinen, die Isoliereinrichtung scheibenförmig auszubilden, da das zu behandelnde Gewebe relativ weich ist.

Weiterhin ist vorzugsweise eine Leitung zum Leisten eines Inertgases zum Gewebe vorgesehen. Das Inertgas kann hierbei als "Schutzgas" derart wirken, dass beim Schneiden und Koagulieren aufgrund der Verdrängung von Sauerstoff keine Verbrennung von Gewebe stattfindet.

Wenn der Bereich zwischen Gewebe und Instrument mit einem Inertgas gefüllt ist, so kann auch eine Koagulation von Gewebe nach dem an sich bekannten Prinzip der "APC" (Argon-Plasma-Koagulation) stattfinden. Hierzu weist die Elektrodeneinrichtung vorzugsweise ein kantig oder spitz geformtes Konzentrationselement zum Erhöhen einer durch eine elektrische Aufladung der Elektrodeneinrichtung erzeugten Feldstärke auf. Diese Konzentrationsvorrichtung kann z.B. einen kantigen Randbereich der Düse umfassen, da bereits durch eine solche Kante eine erhebliche Erhöhung der Feldstärke stattfinden. Alternativ oder zusätzlich kann mindestens eine nadelförmige Anordnung vorgesehen sein, die mit der Elektrodeneinrichtung in elektrisch leitender Verbindung steht, insbesondere mit ihr einstückig ausgebildet ist.

Vorzugsweise ist eine elektrisch isolierende Umhüllungseinrichtung vorgesehen, die derart die Elektrodeneinrichtung verschiebbar umgebend angeordnet ist, dass die Elektrodeneinrichtung in die Umhüllungseinrichtung zurück oder aus ihr herausschiebbar ist. Wenn bei dieser Ausführungsform also die Elektrodeneinrichtung in die Umhüllungseinrichtung zurückgezogen ist, kann eine Gewebeberührung nicht mehr stattfinden und das Instrument ist dann ein reines Wasserstrahl-Chirurgieinstrument. Erst durch Herausschieben aus der Umhüllungseinrichtung wird dann die Elektrodeneinrichtung frei und kann sowohl zum HF-chirurgischen Operieren als auch zum rein mechanischen Präparieren (mittels der entsprechend ausgebildeten Isoliereinrichtung) verwendet werden. Weiterhin wird dann, wenn das Instrument zur "APC-Anwendung" verwendet wird, ein elektrisch leitendes Berühren von Gewebe durch die Elektrodeneinrichtung vermieden. In diesem Fall wird dann also das Instrument zu einer reinen "APC-Sonde".

Um die Handhabung zu vereinfachen, sind bei einer bevorzugten Ausführungsform der Erfindung Anschlageinrichtungen derart angebracht, dass das Zurückziehen bzw. Herausschieben durch die Anschlageinrichtungen begrenzbar ist. Vorzugsweise sind diese Anschlageinrichtungen derart einstellbar, dass das Maß an Herausschieben der Elektrodeneinrichtung aus der Umhüllungseinrichtung ohne erhöhte Sorgfalt des Operateurs durchgeführt werden kann.

Die Versorgung der Elektrodeneinrichtung mit HF-Strom kann über eine gesonderte elektrische Zuleitung erfolgen. Vorzugsweise wird jedoch die Flüssigkeitsstrahleinrichtung mit einem elektrisch leitenden Leitungsabschnitt zum Zuführen der Flüssigkeit und des HF-Stromes ausgebildet, der mit dem Rohrabschnitt dicht und elektrisch leitend verbunden ist. Dadurch kann das Instrument in seinem Raumbedarf minimiert werden, was für endoskopische Anwendungen besonders wichtig ist.

Vorzugsweise besteht der in Gewebekontakt bringbare rohrabschnitt aus einem Material mit einem Schmelzpunkt über etwa 2000 °C, ist also vorzugsweise aus Wolfram gefertigt. Entgegen dem Vorschlag aus der EP 0 280 972 A1 hat es sich gezeigt, dass es bei Verwendung eines Edelstahls zu nicht unerheblichem Abbrand und anderen nachteiligen Effekten kommen kann.

Vorzugsweise wird besonders in diesem Fall der Rohrabschnitt mittels eines Pulverspritzgießverfahrens insbesondere aus Wolfram hergestellt. Derartige Pulverspritzgießverfahren (PIM = Power Injection Molding) sind an sich bekannt und werden nochmals untergliedert in die zwei Fertigungsmethoden "Metallpulverspritzgießen" (MIM = Metal Injection Molding) und "Keramikpulverspritzgießen" (CIM = Ceramic Injection Molding). Durch dieses Verfahren ist es möglich, technisch anspruchsvolle Teile auch im Mikrobereich mittels eines effizienten Fertigungsverfahrens mit ausgezeichneten Toleranzeinhaltungen zu verwirklichen. Dies gelingt, da zwar die Schwindung beim Pulverspritzgießen bei einer Größenordnung von rund 20 % liegt, der Prozess jedoch gut reproduzierbar ist.

Vorzugsweise wird mit dieser Technik auch die Isoliereinrichtung hergestellt, und zwar als Keramik, insbesondere auch Zirkonoxid, Aluminiumoxid, Yttriumoxid oder Mischungen hiervon bestehen kann. Keramiken, deren metallische Komponenten aus der Gruppe IVB (Ti und höher) oder IIIA (Al und höher) entspringen, sind ebenfalls gut geeignet.

Die Isoliereinrichtung kann mittels verschiedener Techniken am distalen Ende des Rohrabschnittes befestigt werden. Besonders bevorzugt und als für sich alleine gesehen erfinderisch wird jedoch ein Verfahren angesehen, bei welchem die Isoliereinrichtung als Keramikkörper ausgebildet wird, wobei ein vorgeformter Grünkörper auf das distale Ende des Rohrabschnittes, dieses wenigstens abschnittsweise umschließend aufgebracht und danach derart gesintert wird, dass durch Schrumpfung der Keramik ein fester Sitz auf dem Rohrabschnitt gewährleistet ist. Es wird also sozusagen ein "Abfallprodukt" bei der Herstellung des Keramikkörpers, nämlich der Schrumpfvorgang, benützt, um gleichzeitig eine feste Verbindung mit dem den Isolierkörper tragenden Rohrabschnitt zu gewährleisten. Es hat sich herausgestellt, dass die Haltekraft einer solchen Verbindung ohne jegliche zusätzliche Maßnahmen besonders hoch ist.

Nachfolgend werden Ausführungsformen der Erfindung anhand von Ausführungsbeispielen näher erläutert. Hierbei zeigen
- - Fig. 1: eine erste Ausführungsform der Erfindung in einem halb-zusammengebauten Zustand,
- - Fig. 2: die Anordnung nach Fig. 1 im zusammengebauten Zustand, wobei die obere Hälfte und die untere Hälfte der Anordnung zwei verschiedene Betriebszustände zeigen,
- - Fig. 3: eine weitere Ausführungsform der Erfindung ähnlich der nach den Fig. 1 und 2,
- - Fig. 4: eine weitere Ausführungsform der Erfindung mit Anschlageinrichtungen,
- - Fig. 5: eine weitere Ausführungsform der Erfindung mit einer Edelgaszuleitung in zwei verschiedenen Betriebszuständen, wie jeweils in der oberen bzw. unteren Hälfte gezeigt,
- - Fig. 6: eine weitere Ausführungsform der Erfindung und
- - Fig. 7: eine Ansicht der Ausführungsform nach Fig. 6 entlang der Linie VII-VII aus Fig. 6.

In der nachfolgenden Beschreibung werden für gleiche und gleich wirkende Teile dieselben Bezugsziffern verwendet.

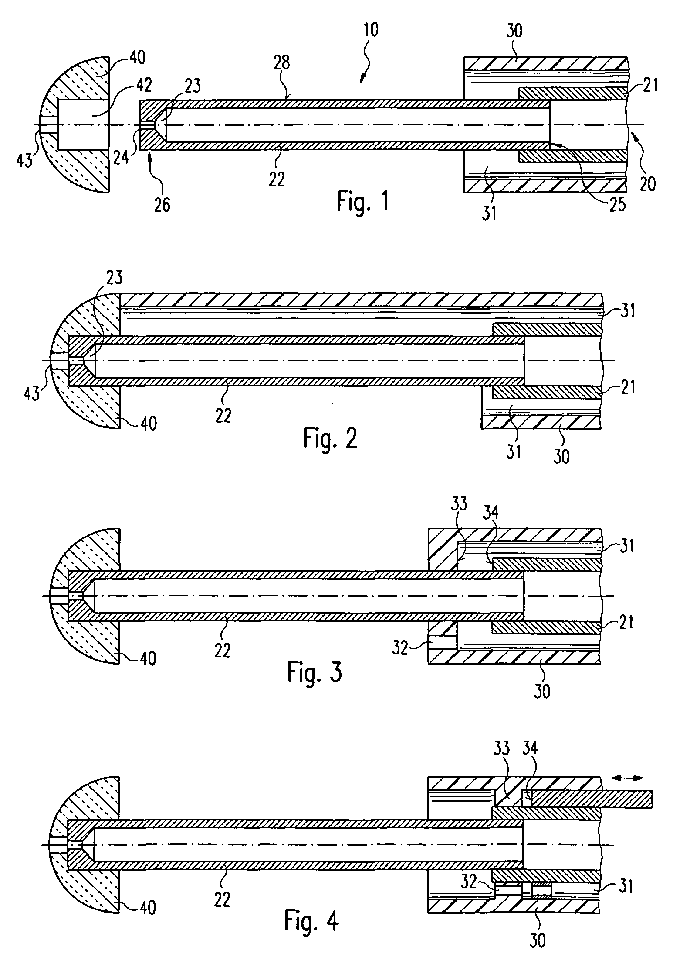

In Fig. 1 ist in einem halb-zusammengebauten Zustand und im Längsschnitt ein Chirurgieinstrument 10 gezeigt, welches eine Flüssigkeitsstrahleinrichtung 20 aufweist. Zur Bildung der Flüssigkeitsstrahleinrichtung 20 ist eine Zufuhrleitung 21 vorgesehen, die mit einer (nicht dargestellten) Pumpeinrichtung zur Erzeugung eines Fluids mit hohem Druck verbunden ist.

Die Zufuhrleitung 21 ist mit einem proximalen Ende 25 eines Rohrabschnittes 22 dicht verbunden. Der Rohrabschnitt 22 besteht ebenso wie Zufuhrleitung 21 aus einem elektrisch leitenden Material, so dass zwischen den beiden Leitungen eine elektrische Verbindung besteht.

Während die Zufuhrleitung 21 aus einem flexiblen (elektrisch leitenden) Material besteht, ist der Rohrabschnitt 22 mittels eines MIM-Verfahrens aus Wolfram gesintert, ist also äußerst steif und abbrandfest.

Der Rohrabschnitt 22 weist an seinem distalen Ende 26 eine Düse 23 auf, die einen relativ scharfkantigen Düsenrand 24 umfasst. Weiterhin ist mit der Bezugsziffer 28 ein Umfangsbereich des Rohrabschnittes 22 bezeichnet, der - wie weiter unten noch beschrieben wird - als Schneidelektrode (gegebenenfalls aber auch zum Koagulieren von Gewebe) Verwendung findet. Somit bildet das Teil 28 eine "Umfangselektrode".

Weiterhin ist eine Isoliereinrichtung 40 vorgesehen, die aus einem keramischen, isolierenden Material (wie oben beschrieben) mittels eines CIM-Verfahrens aufgebaut ist. Die Isoliereinrichtung 40 weist eine Rohraufnahmeöffnung 42 auf, welche zur Aufnahme des distalen Endes 26 des Rohrabschnittes 22 geformt ist. An diese sacklochartig ausgebildete Rohraufnahmeöffnung 42 ist ein Strahlauslass 43 anschließend ausgeformt, der - wenn die Isoliereinrichtung 40 wie in Fig. 2 gezeigt auf den Rohrabschnitt 22 bzw. dessen distales Ende 26 aufgesetzt ist, einem aus der Düse 23 austretenden Wasserstrahl genügend Raum lässt, um hindurch zu treten.

Weiterhin ist eine Umhüllungseinrichtung 30, vorzugsweise ein isolierender, biokompatibler und temperaturbeständiger Kunststoffschlauch vorgesehen, welcher entweder - wie in Fig. 2 in der oberen Hälfte gezeigt - den Rohrabschnitt 22 umgebend oder - wie in Fig. 2 in der unteren Hälfte gezeigt - den Rohrabschnitt 22 freigebend relativ zur Zufuhrleitung 21 und zum Rohrabschnitt. 22 verschiebbar ist.

Der Innenraum bzw. das Lumen der Umhüllungseinrichtung 30 kann hierbei als Leitung 31 benutzt werden, durch welche ein Edelgas, insbesondere Argon in den Bereich zwischen der Umfangselektrode 28 und zu behandelndem Gewebe (nicht gezeigt) geleitet werden kann.

Die Ausführungsformen nach den Fig. 3 und 4 unterscheiden sich dadurch von derjenigen nach den Fig. 1 und 2, dass Anschlageinrichtungen 33, 34 vorgesehen sind, welche ein Herausschieben des Rohrabschnittes 22 aus der Umhüllungseinrichtung 30 nur über einen begrenzten Bereich ermöglichen. Bei der in Fig. 3 gezeigten Ausführungsform ist hierzu die Umhüllungseinrichtung 30 an ihrem Ende bis auf einen Durchlass für den Rohrabschnitt 23 geschlossen. Diese "Endwand" bildet den ersten Anschlag 33. Der zweite Anschlag 34 wird durch die Zufuhrleitung 21 bzw. deren distales Ende gebildet. Um nach wie vor einen Gasausstrom aus der Leitung 31 sicherzustellen, ist eine Ausströmöffnung 32 in der "Endwand" der Umhüllungseinrichtung 30 vorgesehen.

Bei der in Fig. 4 gezeigten Ausführungsform ist eine gesonderte Verengungsstelle als erster Anschlag 33 in der Umhüllungseinrichtung 30 vorgesehen. Der zweite Anschlag 34 wird durch ein Zusatzteil gebildet, welches auf der Zufuhrleitung 21 sitzt. Dieser zweite Anschlag 34 ist (wie mit dem Doppelpfeil in Fig. 4 angedeutet) derart verschiebbar, dass das Maß, um welches der Rohrabschnitt 22 aus der Umhüllungseinrichtung 30 herausgeschoben werden kann, einstellbar ist. Es sei hier bemerkt, dass die in Fig. 4 gezeigten Bemessungen nicht vollständig den optimalen Verhältnissen entsprechen.

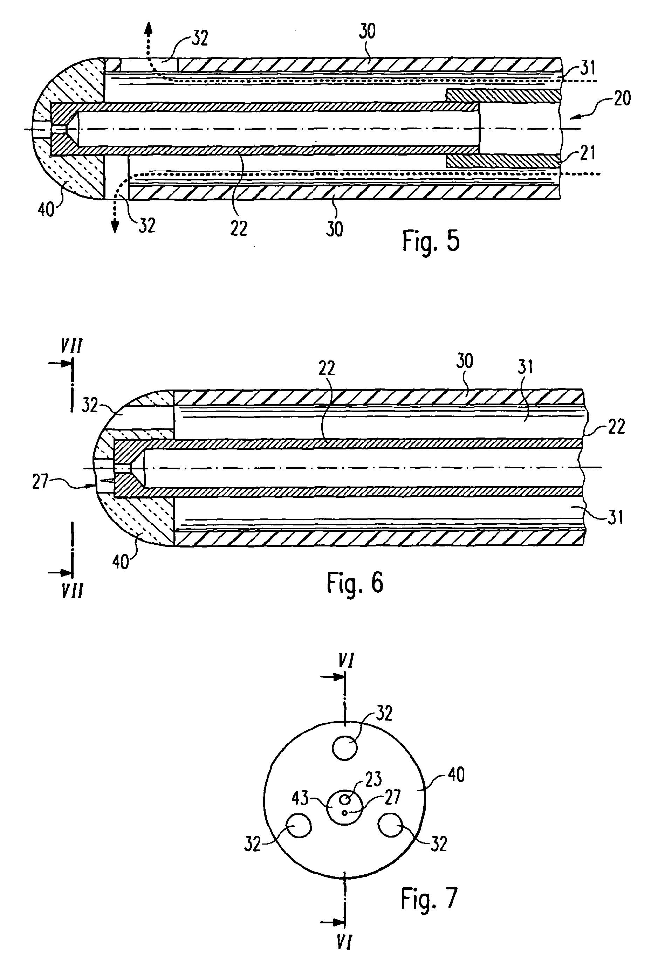

Die in Fig. 5 gezeigte Variante unterscheidet sich von den zuvor gezeigten Ausführungsformen durch eine zusätzlich dargestellte Ausströmöffnung 32 (obere Hälfte der Fig. 5), durch welche Edelgas, das durch die Leitung 31 strömt, seitwärts (wie mit der punktierten Linie angedeutet) ausströmen kann. Bei der in Fig. 5 in der unteren Zeichnung zur Hälfte gezeigten Variante ist die Anordnung so getroffen, dass die Ausströmöffnung 32 durch einen Spalt zwischen einem distalen Endrand der Umhüllungseinrichtung 30 und der Isoliereinrichtung 40 gebildet wird (auch hier ist der Gasstrom mit einem punktierten Pfeil angedeutet).

Die in Fig. 6 gezeigte Ausführungsform der Erfindung unterscheidet sich von der nach Fig. 5 zunächst dadurch, dass die Ausströmöffnung 32 zum Ausstrom eines durch die Leitung 31 strömenden Edelgases durch drei Löcher gebildet ist (siehe Fig. 7), welche in der Isoliereinrichtung 40 angebracht sind.

Weiterhin unterscheidet sich die Ausführungsform nach den Fig. 6 und 7 dadurch, dass auf der distalen Endfläche des Rohrabschnittes 22 ein kegelförmiges Konzentrationselement 27 angebracht ist, das mit dem Rohrabschnitt 22 einstückig ausgebildet ist (jedoch auch eingesetzt sein kann).

Mit dem so aufgebauten Instrument können drei Funktionen verwirklicht werden.

Zum Einen kann eine Wasserstrahlchirurgie mittels Wasserstrahleinrichtung durchgeführt werden. Zum Zweiten kann ein HF-chirurgischer Schnitt durchgeführt werden, wobei ein entsprechender hochfrequenter Strom dem Rohrabschnitt 22 zugeführt und dessen Umfang, also die Umfangselektrode 28 mit einem Gewebe in Berührung gebracht wird. Dabei kann die Isoliereinrichtung 40 gleichzeitig als Präparationsinstrument zum mechanischen Präparieren (z.B. Öffnen) von Gewebe verwendet werden. Zum Dritten kann eine Plasma-Koagulation durchgeführt werden, wobei durch die Leitung 31 und die Ausströmöffnungen 32 ein Edelgas so zugeführt wird, dass es einen Bereich zwischen einem zu koagulierenden Gewebe und dem distalen Ende des Rohrabschnittes 22 füllt. Wenn dann eine entsprechend hohe hochfrequente Spannung über die Zufuhrleitung 21 und den Rohrabschnitt 22 zugeführt wird, so bildet sich zwischen dem Gewebe und denjenigen Stellen des Rohrabschnittes 22 eine hochfrequente Entladung mit Bildung eines entsprechenden Plasmas aus, an denen die Feldstärke besonders hoch ist. Die elektrische Feldstärke wird an solchen Stellen besonders hoch, die kantig oder spitz ausgebildet sind (dies ist unter dem Stichwort Spitzenentladung bekannt). Im vorliegenden Fall bei den Ausführungsformen nach den Fig. 1 bis 5 ist dies im Allgemeinen der Düsenrand 24, da alle anderen "kantigen Teile" des Rohrabschnittes 22 entweder durch die Isoliereinrichtung 40 abgedeckt oder zu weit entfernt vom Gewebe sind. Eine weitere Erhöhung der Feldstärke wird bei dem in den Fig. 6 und 7 gezeigten Ausführungsbeispiel durch das spitzenförmige Konzentrationselement 27 gebildet. Um dies zu ermöglichen, ist in der Isoliereinrichtung 40 ein vergrößerter Strahlauslass 43 vorgesehen, der derart groß ausgebildet ist, dass sowohl das Konzentrationselement 27 frei ist als auch genügend Raum zum Austritt des Flüssigkeitsstrahls aus der Düse 23 ermöglicht wird.

Aus Obigem ergibt sich, dass mit der vorliegenden Erfindung ein hochkompaktes Kombinations- oder Multifunktionsinstrument geschaffen wird, das für endoskopische Operationen besonders geeignet ist.

### Bezugszeichenliste

- 10: Chirurgieinstrument
- 20: Flüssigkeitsstrahleinrichtung
- 21: Zufuhrleitung
- 22: Rohrabschnitt
- 23: Düse
- 24: Düsenrand
- 25: proximales Ende
- 26: distales Ende
- 27: Konzentrationselement
- 28: Umfangelektrode
- 30: Umhüllungseinrichtung
- 31: Leitung
- 32: Ausströmöffnung
- 33: erster Anschlag
- 34: zweiter Anschlag
- 40: Isoliereinrichtung
- 42: Rohraufnahmeöffnung
- 43: Strahlauslass

## Patentansprüche

1. Endoskopisches Chirurgieinstrument, umfassend
eine Flüssigkeitsstrahleinrichtung (20) mit einem Rohrabschnitt (22) mit einer Düse (23) an einem distalen Ende (26) für eine Dissektion und/oder nadellose Injektion mittels einer Flüssigkeit und eine Elektrodeneinrichtung (28) zum Schneiden und/oder Koagulieren eines Gewebes mittels HF-Strom,
**dadurch gekennzeichnet, dass**
der Rohrabschnitt (22) eine Elektrodeneinrichtung (28) bildet, und dass am distalen Ende (26) des Rohrabschnittes (22) eine Isoliereinrichtung (40) derart angebracht ist, dass das Gewebe nur mit einem Umfangsbereich (28) und nicht mit dem distalen Ende (26) des Rohrabschnittes (22) ein elektrisch leitende Berührung bringbar ist.

2. Endaskopisches Chirurgieinstrument nach Anspruch 1,
**dadurch gekennzeichnet, dass**
die Isoliereinrichtung (40) als mechanische Präparationseinrichtung zum Präparieren von Gewebe ausgebildet ist.

3. Endoskopisches Chirurgieinstrument nach einem der vorhergehenden Ansprüche,
**dadurch gekennzeichnet, dass**
eine Leitung (31) zum Leiten eines Inertgases zum Gewebe vorgesehen ist.

4. Endoskopisches Chirurgieinstrument nach einem der vorhergehenden Ansprüche**,**
**dadurch gekennzeichnet, dass**
die Elektrodeneinrichtung ein kantig oder spitz geformtes Konzentrationselement (27) zum Erhöhen einer durch eine elektrische Aufladung der Elektrodeneinrichtung (28) erzeugten Feldstärke umfasst.

5. Endoskopische Chirurgieinstrument nach einem der vorhergehenden Ansprüche,
**gekennzeichnet durch**
eine elektrisch Isolierende Umhüllungseinrichtung (30), die derart die Elektrodeneinrichtung (28) verschiebbar umgebend angeordnet ist, dass die Elektrodeneinrichtung (28) in die Umhüllungseinrichtung (30) zurück oder aus ihr herausschiebbar ist.

6. Endoskopisches Chirurgieinstrument nach Anspruch 5,
**gekennzeichnet durch**
Anschlageinrichtungen (33, 34), welche derart angebracht sind, dass das Zurückziehen/Herausschieben **durch** die Anschlageinrichtungen (33, 34) begrenzbar ist.

7. Endoskopisches Chirurgieinstrument nach Anspruch 6,
**dadurch gekennzeichnet, dass**
die Anschlageinrichtungen (34) einstellbar sind.

8. Endoskopisches Chirurgieinstrument nach einem der vorhergehenden Ansprüche,
**dadurch gekennzeichnet, dass**
die Flüssigkeitsstrahleinrichtung (20) einen elektrisch leitenden Leitungsabschnitt (21) zum Zuführen der Flüssigkeit und des HF-Stromes umfasst, der mit dem Rohrabschnitt (22) dicht und elektrisch leitend verbunden ist.

9. Endoskopisches Chirurgleinstrument nach einem der vorhergehenden Ansprüche,
**dadurch gekennzeichnet, dass**,
der Rohrabschnitt (22) aus einem Material mit einem Schmelzpunkt über etwa 2000 °C, vorzugsweise aus Wolfram gefertigt ist.

10. Endoskopisches Chirurgleinstrument nach einem der vorhergehenden Ansprüche,
insbesondere nach Anspruch 9,
**dadurch gekennzeichnet, dass**
der Rohrabschnitt (22) mittels eines Pulverspritzgleßverfahrens insbesondere aus Wolfram hergestellt ist.

11. Endoskopisches Chirurgieinstrument nach einem der vorhergehenden Ansprüche,
**dadurch gekennzeichnet, dass**
die Isoliereinrichtung (40) mittels eines Pulverspritzgießverfahrens insbesondere aus einer Keramik, insbesondere aus Zirkonoxid, Aluminiumoxid, Yttriumoxid oder Mischungen hiervon oder aus Keramiken gefertigt ist, deren metallische Komponenten aus der Gruppe IVB (Ti und höher) oder IIIA (Al und höher) entspringen.

12. Verfahren zum Herstellen eines endoskopischen Chirurgieinstrumentes, nach Anspruch 1
**dadurch gekennzeichnet, dass**
die Iscliereinrichtung als Keramik ausgebildet wird, wobei ein vorgeformter Grünkörper auf das distale Ende des Rohrabschnittes, dieses weinigstens abschnittsweise umschließend aufgebracht und danach derart gesintert wird, dass durch Schrumpfung der Keramik ein fester Sitz auf dem Rohrabschnitt gewährleistet ist.

## Claims

1. Endoscopic surgical instrument comprising a liquid spray device (20) with a tube section (22) with a nozzle (23) at a distal end (26) for a dissection and/or needle-less injection by means of a liquid and
an electrode device (28) for cutting and/or coagulating a tissue by means ofHF current, **characterised in that**
the tube section (22) forms an electrode device (28), and that an isolation device (40) is attached to the distal end (26) of the tube section (22) in such a manner that the tissue can only be brought into electrically conductive contact with a peripheral region (28) and not with the distal end (26) of the tube section (22).

2. Endoscopic surgical instrument according to claim 1, **characterised in that** the isolation device (40) is configured as a mechanical preparation device for preparing tissue.

3. Endoscopic surgical instrument according to claim 1, **characterised in that** a conduit (31) is provided to conduct an inert gas to the tissue.

4. Endoscopic surgical instrument according to one of the preceding claims,
**characterised in that**
the electrode device comprises an angular or pointed concentration element (27) for increasing a field intensity generated by an electrical charging of the electrode device (28).

5. Endoscopic surgical instrument according to one of the preceding claims,
**characterised by**
an electrically conductive enveloping device (30), which is arranged to displaceably enclose the electrode device (28) in such a manner that the electrode device (28) can be slid back into or slid out of the enveloping device (30).

6. Endoscopic surgical instrument according to claim 5, **characterised by** stop devices (33, 34), which are attached in such a manner that the retraction/extension can be restricted by the stop devices (33, 34).

7. Endoscopic surgical instrument according to claim 6, **characterised in that** the stop devices (34) are adjustable.

8. Endoscopic surgical instrument according to one of the preceding claims,
**characterised in that**
the liquid spray device (20) comprises an electrically conductive conduit section (21) for supplying the liquid and the HF current, which is connected to the tube section (22) in a sealed and electrically conductive manner.

9. Endoscopic surgical instrument according to one of the preceding claims,
**characterised in that**
the tube section (22) is made from a material with a melting point above about 2000°C, preferably tungsten.

10. Endoscopic surgical instrument according to one of the preceding claims,
**characterised in that**
the tube section (22) is produced by means of a powder injection moulding process in particular from tungsten.

11. Endoscopic surgical instrument according to one of the preceding claims,
**characterised in that**
the isolation device (40) is produced by means of a powder injection moulding process in particular from a ceramic, in particular from zirconium oxide, aluminium oxide, yttrium oxide or mixtures thereof or from ceramics whose metallic components come from group IVB (Ti and higher) or IIIA (Al and higher).

12. Process for the production of an endoscopic surgical instrument according to claim 1,
**characterised in that**
the isolation device is made from ceramic, wherein a preformed green compact is placed onto the distal end of the tube section to surround this at least in sections and is then sintered in such a manner as to assure a firm fit on the tube section by contraction of the ceramic.

## Revendications

1. Instrument chirurgical endoscopique comprenant un dispositif à jet de liquide (20) avec une portion tubulaire (22) équipée d'une buse (23) à une extrémité distale (26) pour une dissection et/ou une injection sans aiguille au moyen d'un liquide et une dispositif d'électrode (28) pour couper et/ou coaguler un tissu au moyen d'un courant HF, **caractérisé en ce que** la portion tubulaire (22) forme un dispositif d'électrode (28), et **en ce qu'**un dispositif isolant (40) est monté à l'extrémité distale (26) de la portion tubulaire (22), de façon que le tissu puisse être mis en contact électriquement conducteur seulement avec une région périphérique (28) et non avec l'extrémité distale (26) de la portion tubulaire (22).

2. Instrument chirurgical endoscopique selon la revendication 1, **caractérisé en ce que** le dispositif isolant (40) est réalisé sous la forme d'un dispositif de préparation mécanique pour la préparation de tissus.

3. Instrument chirurgical endoscopique selon une des revendications précédentes, **caractérisé en ce qu'**une conduite (31) pour conduire un gaz inerte vers le tissu est prévue.

4. Instrument chirurgical endoscopique selon une des revendications précédentes, **caractérisé en ce que** le dispositif d'électrode comprend un élément de concentration (27) de forme anguleuse ou pointue, destiné à augmenter une intensité de champ produite par une charge électrique du dispositif d'électrode (28).

5. Instrument chirurgical endoscopique selon une des revendications précédentes, **caractérisé par** un dispositif de recouvrement (30) électriquement isolant qui est disposé de manière à entourer le dispositif d'électrode de façon mobile, de façon que le dispositif d'électrode (28) puisse être rétracté dans le dispositif de recouvrement (30) ou sorti dudit dispositif de recouvrement (30).

6. Instrument chirurgical endoscopique selon la revendication 5, **caractérisé par** des dispositifs de butée (33, 34) qui sont disposés de façon que la rétraction et l'extraction soient limitées par les dispositifs de butée (33, 34).

7. Instrument chirurgical endoscopique selon la revendication 6, **caractérisé en ce que** les dispositifs de butée (34) sont réglables.

8. Instrument chirurgical endoscopique selon une des revendications précédentes, **caractérisé en ce que** le dispositif à jet de liquide (20) comprend, pour l'amenée du liquide et du courant HF, une portion de conduite (21) électriquement conductrice qui est reliée de manière étanche et électriquement conductrice à la portion tubulaire (22).

9. Instrument chirurgical endoscopique selon une des revendications précédentes, **caractérisé en ce que** la portion tubulaire (22) est réalisée dans un matériau ayant un point de fusion supérieur à environ 2 000 °C, de préférence en tungstène.

10. Instrument chirurgical endoscopique selon une des revendications précédentes, en particulier selon la revendication 9, **caractérisé en ce que** la portion tubulaire (22) est réalisée au moyen d'un procédé de moulage par injection de poudre, en particulier en tungstène.

11. Instrument chirurgical endoscopique selon une des revendications précédentes, **caractérisé en ce que** le dispositif isolant (40) est réalisé au moyen d'un procédé de moulage par injection de poudre, en particulier en céramique, en particulier en oxyde de zirconium, en oxyde d'aluminium, en oxyde d'yttrium ou dans des mélanges de ceux-ci ou en céramiques dont les composants métalliques proviennent du groupe IVB (Ti et supérieur) ou IIIA (Al et supérieur).

12. Procédé de fabrication d'un Instrument chirurgical endoscopique selon la revendication 1, **caractérisé en ce que** le dispositif isolant est réalisé sous la forme d'une céramique, un corps de base préformé étant placé sur l'extrémité distale de la portion tubulaire, en entourant celle-ci au moins en partie, et ensuite fritté, de façon qu'une fixation solide sur la portion tubulaire soit assurée par retrait de la céramique.
